# EUROPEAN PATENT SPECIFICATION

(11) **EP 3 322 709 B1**
(45) Date of publication and mention of the grant of the patent: **25.11.2020**
(21) Application number: 16748052.4
(22) Date of filing: 14.07.2016
(51) Int. Cl.: C07D 487/04

(54) **CRYSTALLINE FORMS OF (3R)-3-CYCLOPENTYL-3-[4-(7H-PYRROLO[2,3-D]PYRIMIDIN-4-YL)PYRAZOL-1-YL]PROPANENITRILE SALTS AND PREPARATION THEREOF**
KRISTALLINE FORMEN VON (3R)-3-CYCLOPENTYL-3-[4-(7H-PYRROLO[2,3-D]PYRIMIDIN-4-YL)PYRAZOL-1-YL]PROPAN NITRILE SALZEN UND IHRE HERSTELLUNG
FORMES CRYSTALLINES DES SELS DU (3R)-3-CYCLOPENTYL-3-[4-(7H-PYRROLO[2,3-D]PYRIMIDIN-4-YL)PYRAZOL-1-YL]PROPANENITRILE ET LEUR PREPARATION

(30) Priority: 14.07.2015 CZ 20150496
(43) Date of publication of application: 23.05.2018
(73) Proprietor: Zentiva K.S., 102 37 Praha 10 (CZ)
(72) Inventor: KISS, Violetta, 7042 Palfa (HU); TOZICKOVA, Hana, 155 00 Praha 5 (CZ); TIEGER, Eszter, 9963 Magyarlak (HU); DAMMER, Ondrej, 253 01 Hostivice (CZ); GURGUT, Tomas, 142 00 Praha 12 (CZ)
(74) Representative: Ellis, Robin Patrick
(86) International application number: PCT/CZ2016/000077
(87) International publication number: WO 2017/008772

(56) References cited:
- WO-A1-2016/026975
- WO-A1-2016/074650
- WO-A2-2008/157208

## Description

### Technical Field

The present invention relates to crystalline forms of (3R)-3-cyclopentyl-3-[4-(7H-pyrolo[2,3-d]pyrimidin-4-yl)pyrazol-1-yl]propanenitrile salts of Formula I, characterized in that the acid component HX is hydrobromic acid in a crystalline form having an X-ray powder diffraction pattern with characteristic peaks at about 4.3; 14.8; 18.9; 20.3; 22.5 and 25.6 ± 0.2° 2-theta.

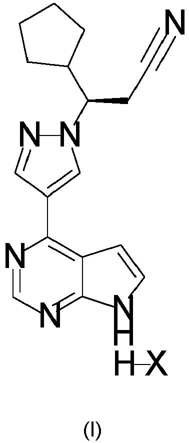

The invention also relates to processes for the preparation of the crystal modifications as well as pharmaceutical compositions .

### Background Art

(3R)-3-Cyclopentyl-3-[4-(7H-pyrrolo[2,3-d]pyrimidin-4-yl)pyrazol-1-yl]propanenitrile compound which is also known as ruxolitinib (CAS no.: 941678-49-5) has a selective inhibitor activity on the Janus Associated Kinase 1 (JAK1) and Janus Associated Kinase 2 (JAK2) enzymes. It is a drug indicated for the treatment of intermediate or high-risk myelofibrosis, which is a type of bone marrow cancer.

The enzymes Janus Associated Kinase 1 (JAK1) and Janus Associated Kinase 2 (JAK2) are non-receptor tyrosine kinases that mediate the signals via the JAK-STAT pathway. Cytokines play important roles in the control of the cell growth and the immune response. More specifically, Janus Associated Kinases are phosphorylate activated cytokine receptors recruiting STAT transcription factors which modulate gene transcription.

WO2007070514 describes protein kinase inhibitors with valuable pharmacological effect in the treatment of related diseases. One example of the compounds disclosed is (3R)-3-cyclopentyl-3-[4-(7H-pyrrolo[2,3-d]pyrimidin-4-yl)pyrazol-1-yl]propanenitrile. Preparation of the base is also described.

Salts of (3R)-3-cyclopentyl-3-[4-(7H-pyrrolo[2,3-d]pyrimidin-4-yl)pyrazol-1-yl]propanenitrile prepared with maleic acid, sulphuric acid and phosphoric acid are disclosed in WO2008157208. Following WO2010083283 discloses the process for the preparation of ruxolitinib phosphate with good yield and excellent chemical and optical purity.

Many pharmaceutical solid compounds can exist in various crystalline forms regarded as polymorphs and hydrates/solvates having different crystal units and hence different physico-chemical properties including melting point, solubility, dissolution rate and finally, bioavailability. In order to distinguish the distinct solid phases of a compound several solid state analytical techniques can be used, e.g. X-Ray Powder Diffraction, solid state NMR and Raman spectroscopy, thermoanalytical methods.

Discovery of new solid phases (polymorphs, solvates and hydrates) of an active pharmaceutical compound offers the opportunity to select the appropriate modification having desirable physicochemical properties and processability and improve the characteristics of the pharmaceutical product. For this reason there is an explicit need for new solid forms (polymorphs, solvates, hydrates) of ruxolitinib and salts thereof especially in the crystalline form.

WO 2008/157208 A2 relates to salts of the Janus kinase inhibitor (R)-3-(4-(7H-PYRROLO(2,3-D)PYRIMIDIN-4-YL)-1H-PYRAZOL-1-YL)-3-CYCLOPENTYLPROPANENITRILE.

WO 2016/026975 A1 relates to a salt of (R)-3-(4-(7H-PYRROLO [2,3-D]PYRIMIDIN-4-YL)-LH-PYRAZOL-L-YL)-3-CYCLOPENTYLPROPANENITRILE with benzenesulfonic acid, WO 2016/074650 A1 relates to salts of (3R)-3-CYCLOPENTYL-3-[4-(7H-PYRROLO[2,3-D]PYRIMIDIN-4-YL)PYRAZOL-1-YL]PROPANENITRILE.

### Disclosure of Invention

The object of the present invention is to provide novel crystalline salts comprising (3R)-3-cyclopentyl-3-[4-(7H-pyrrolo[2,3-d]pyrimidin-4-yl)pyrazol-1-yl]propanenitrile of Formula I and at least one acid component (HX) suitable for oral administration which meet the pharmaceutical requirements 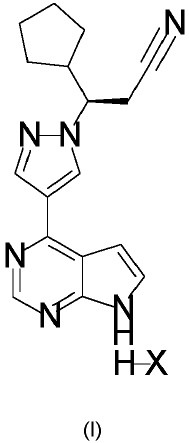 wherein HX is hydrobromic acid.

The solid forms are characterized by a variety of solid state analytical data, including for example X-ray powder diffraction pattern (XRPD) and differential scanning calorimetry (DSC) curve.

Provided is the Crystal modification 5 of (3R)-3-cyclopentyl-3-[4-(7H-pyrrolo[2,3-d]pyrimidin-4-yl)pyrazol-1-yl]propanenitrile hydrobromic acid salt of Formula I, wherein X represents Br, having an X-ray powder diffraction pattern comprising characteristic peaks at about 4.3; 14.8; 18.9; 20.3; 22.5 and 25.6 ± 0.2° 2-theta measured by CuKα radiation. In some embodiments the Crystal modification 5 is characterised by differential scanning calorimetry curve having a melting process with Tₒₙₛₑₜ=175.2°C and Tₚₑₐₖ=196.2°C. In some embodiments the Crystal modification 5 is characterised by the thermal gravimetric curve having a 0.47% weight loss in the range of 25°C to 190°C.

It should be understood that relative intensities can vary depending on a number of factors, including sample preparation, mounting, and the instrument and analytical procedure and settings used to obtain the spectrum.

Provided is a process for the preparation of the Crystal modification 5, wherein ruxolitinib hydrobromic acid is suspended in ethyl acetate at room temperature, preferably the suspension is stirred for about 6 weeks. In some embodiments the process for the preparation of the Crystal modification 5 further comprises the steps of: a) suspending ruxolitinib hydrobromic acid salt in ethyl acetate at room temperature; b) stirring the suspension of step a) at room temperature for about 6 weeks; c) isolating the Crystal modification 5 of ruxolitinib hydrobromic acid salt and d) optionally, drying of the product of step c) at laboratory condition until the constant weight of the product is reached.

Provided is another process for the preparation of the Crystal modification 5 wherein ruxolitinib free base is dissolved in an organic solvent by heating of the system to a temperature 50°C followed by the addition of the counterion, then kept at the temperature of 50°C for additional 1 hour and finally cooled back to room temperature.

In some embodiments the process for the preparation of the Crystal modification 5 further comprises the steps of: a) dissolution of ruxolitinib free base in a polar aprotic solvent selected from the group consisting of: acetone, acetonitrile, ethyl acetate, butyl acetate, tetrahydrofuran or a mixture thereof, more preferably in acetonitrile at the temperature of 50°C; b) drop-wise addition of the hydrobromic acid 48% aqueous solution; c/ stirring the solution of the step b) at 50°C for additional 1 hour; d) cooling the solution of the step c) to room temperature; e) keeping the solution of the step d) for about 16 hours at room temperature while precipitation occurred; ) isolating the ruxolitinib hydrobromic acid salt in Crystal modification 5 and g) optionally, drying of the product of step f) at laboratory condition until the constant weight of the product is reached.

In another embodiment the present invention further relates to the use of the Crystal modification 5 of (3R)-3-cyclopentyl-3-[4-(7H-pyrrolo[2,3-d]pyrimidin-4-yl)pyrazol-1-yl]propanenitrile hydrobromic acid salt of Formula I, wherein X represents Br, for the preparation of a pharmaceutical composition.

The present invention further relates to a pharmaceutical formulations containing one or more solid forms of (3R)-3-cyclopentyl-3-[4-(7H-pyrrolo[2,3-d]pyrimidin-4-yl)pyrazol-1-yl]propanenitrile hydrobromic acid of Crystal modification 5) and a pharmaceutically acceptable carrier for the use thereof for the treatment of cancer.

The present invention further relates to a pharmaceutical formulations containing the modifications of (3R)-3-cyclopentyl-3-[4-(7H-pyrrolo[2,3-d]pyrimidin-4-yl)pyrazol-1-yl]propanenitrile hydrobromic acid salt and the use thereof for the treatment of cancer.

It has now surprisingly been found that the compound of Formula I wherein HX represents hydrobromic acid. These solid phase modifications, referred to herein as Crystal modification 5 of the (3R)-3-cyclopentyl-3-[4-(7H-pyrrolo[2,3-d]pyrimidin-4-yl)pyrazol-1-yl]propanenitrile hydrobromic acid salt have different physico-chemical properties. Each solid phases were identified by characteristic X-Ray Powder diffractograms and Raman spectra and differ in their Differential Scanning Calorimetry and Thermal Gravimetric Analysis curves, too. Discovery of new crystalline solid phases of (3R)-3-cyclopentyl-3-[4-(7H-pyrrolo[2,3-d]pyrimidin-4-yl)pyrazol-1-yl]propanenitrile (Ruxolitinib) offers the opportunity to select the appropriate modification having desirable physicochemical properties and processability and improve the characteristics of the pharmaceutical product. For this reason there is an explicit need for new solid forms (polymorphs, solvates, hydrates) of ruxolitinib and salts thereof especially in the their crystalline form as this is much more easily to be prepared, separated and purified and also used as the final form of the active pharmaceutical ingredient in the finished product.

### Brief description of Drawings

The figures depict the following spectra, patterns and curves the various solid phases prepared according to the present invention.
**Figure 1** is an XRPD pattern of the Crystal modification 5 of (3R)-3-cyclopentyl-3-[4-(7H-pyrrolo[2,3-d]pyrimidin-4-yl)pyrazol-1-yl]propanenitrile hydrobromic acid salt;
**Figure 2** is an FTIR spectra of the Crystal modification 5 of (3R)-3-cyclopentyl-3-[4-(7H-pyrrolo[2,3-d]pyrimidin-4-yl)pyrazol-1-yl]propanenitrile hydrobromic acid salt;
**Figure 3** is a Raman spectra of the Crystal modification 5 of (3R)-3-cyclopentyl-3-[4-(7H-pyrrolo[2,3-d]pyrimidin-4-yl)pyrazol-1-yl]propanenitrile hydrobromic acid salt;
**Figure 4** is a DSC curve of the Crystal modification 5 of (3R)-3-cyclopentyl-3-[4-(7H-pyrrolo[2,3-d]pyrimidin-4-yl)pyrazol-1-yl]propanenitrile hydrobromic acid salt;
**Figure 5** is a TGA curve of the Crystal modification 5 of (3R)-3-cyclopentyl-3-[4-(7H-pyrrolo[2,3-d]pyrimidin-4-yl)pyrazol-1-yl]propanenitrile hydrobromic acid salt;

### Detailed description of the invention

The following description is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles described herein may be applied to other examples and applications without departing from the spirit and scope of the various embodiments. Therefore, the various embodiments are not intended to be limited to the examples described herein and shown, but are to be accorded the scope consistent with the claims.

The aim of the present invention is to provide novel crystalline forms of (3R)-3-cyclopentyl-3-[4-(7H-pyrolo[2,3-d]pyrimidin-4-yl)pyrazol-1-yl]propanenitrile salts of Formula I, 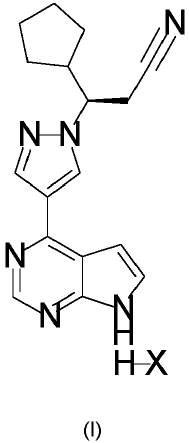 wherein HX is hydrobromic acid, with advantageous properties for pharmaceutical use regarding the physico-chemical properties and can be produced in a reproducible manner even in industrial scale. The invention also relates to the processes for the preparation thereof as well as said use thereof in pharmaceutically acceptable compositions.

Variations in the crystal structure of ruxolitinib salts may affect the dissolution rate (which may affect bioavailability etc.), manufacturability (*e.g.*, ease of handling, ability to consistently prepare doses of known strength) and stability (*e.g.*, thermal stability, shelf life, etc.) of a pharmaceutical drug product, particularly when formulated in a solid oral dosage form (*e.g.*, in a form of a tablet). The therapeutic use and manufacturing of ruxolitinib involves the development of a new solid form of ruxolitinib salts that is more bioavailable and stable.

The term "modification, modifications" of ruxolitinib, as used in this document, is synonymous to terms "solid state form, solid phase modification" of ruxolitinib and includes crystalline modifications, hydrates and solvates of ruxolitinib.

The term "crystal modification" of ruxolitinib, as used in this document, is synonymous to commonly used expressions "polymorphic form" or "crystalline form" of ruxolitinib.

The use of the term "about" includes and describes the value or parameter per se. For example, "about x" includes and describes "x" per se. In some embodiments, the term "about" when used in association with a measurement, or used to modify a value, a unit, a constant, or a range of values, refers to variations of +/- 20 percent, preferably +/- 10 percent and even more preferably +/- 5 percent.

The term "substantially" or "substantially free/pure" with respect to a particular solid form of a compound means that the polymorphic form contains about less than 30 percent, about less than 20 percent, about less than 15 percent, about less than 10 percent, about less than 5 percent, or about less than 1 percent by weight of impurities. In other embodiments, "substantially" or "substantially free/pure" refers to a substance free of impurities. Impurities may, for example, include by-products or left over reagents from chemical reactions, contaminants, degradation products, other polymorphic forms, water, and solvents.

It has now been surprisingly found that the above-mentioned crystalline salts of (3R)-3-cyclopentyl-3-[4-(7H-pyrrolo[2,3-d]pyrimidin-4-yl)pyrazol-1-yl] with at least one acid component HX, wherein HX is hydrobromic acid, can be prepared and have not been described in the literature yet and no solid state analytical data (X-Ray Powder Diffraction patterns, Single-Crystal X-Ray Diffraction data etc.) serving to characterize the crystalline phases have been provided.

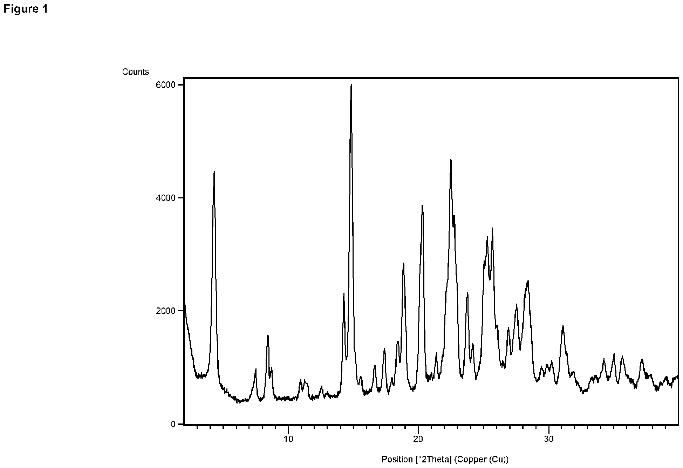

The Crystal modification 5 of (3R)-3-cyclopentyl-3-[4-(7H-pyrrolo[2,3-d]pyrimidin-4-yl)pyrazol-1-yl]propanenitrile hydrobromic acid salt according to the invention has the characteristic XRPD pattern as shown in **Figure 1**. XRPD pattern was recorded on an X-Ray Powder Diffractometer (X'PERT PRO MPD PANalytical). The Crystal modification 5 of (3R)-3-cyclopentyl-3-[4-(7H-pyrrolo[2,3-d]pyrimidin-4-yl)pyrazol-1-yl]propanenitrile hydrobromic acid salt exhibits the following diffraction peaks in XRPD pattern, see **Table 1**, below:

**Table 1**

| **Pos. [°2Th.]** | **d-spacing [Å]** | **Rel. Int. [%]** |
|---|---|---|
| 4.27 | 20.679 | 65.0 |
| 7.43 | 11.885 | 8.4 |
| 8.40 | 10.519 | 19.4 |
| 14.26 | 6.208 | 28.4 |
| 14.82 | 5.974 | 100.0 |
| 16.63 | 5.325 | 7.9 |
| 17.37 | 5.103 | 13.0 |
| 18.40 | 4.819 | 14.7 |
| 18.86 | 4.701 | 38.7 |
| 20.32 | 4.366 | 51.6 |
| 21.35 | 4.158 | 7.2 |
| 22.49 | 3.951 | 63.1 |
| 23.68 | 3.754 | 20.7 |
| 24.15 | 3.682 | 9.5 |
| 25.27 | 3.522 | 36.3 |
| 25.64 | 3.471 | 42.0 |
| 26.88 | 3.314 | 13.0 |
| 27.48 | 3.243 | 20.2 |
| 28.43 | 3.137 | 24.4 |
| 29.42 | 3.033 | 3.9 |
| 29.86 | 2.990 | 4.6 |
| 30.21 | 2.956 | 5.5 |
| 31.08 | 2.875 | 17.6 |
| 34.21 | 2.619 | 7.3 |
| 34.95 | 2.565 | 8.2 |
| 35.67 | 2.515 | 8.3 |
| 37.14 | 2.419 | 8.7 |

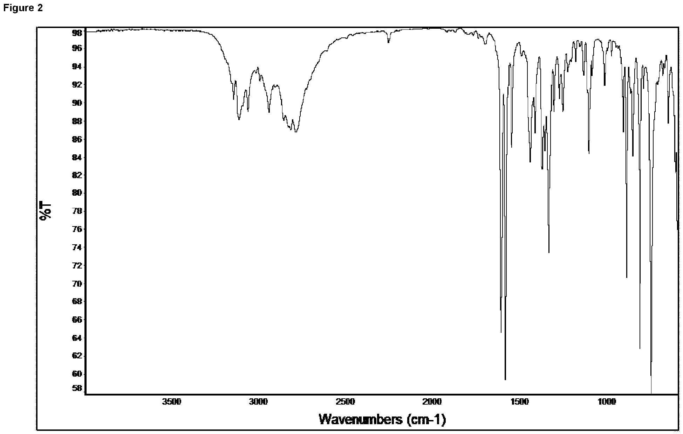

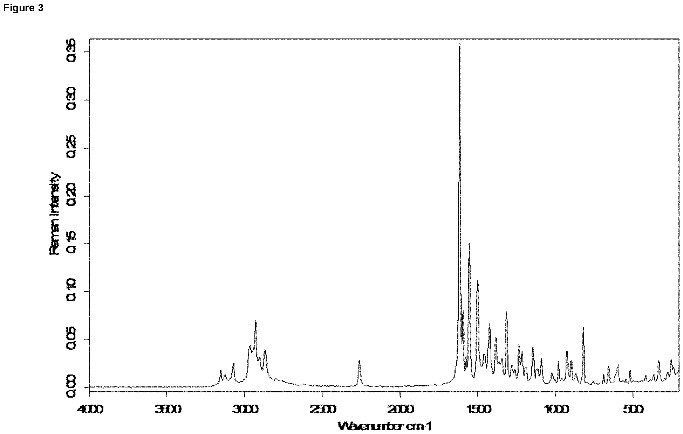

The Crystal modification 5 of (3R)-3-cyclopentyl-3-[4-(7H-pyrrolo[2,3-d]pyrimidin-4-yl)pyrazol-1-yl]propanenitrile hydrobromic acid salt can be characterized by FTIR and Raman spectroscopy. **Figure 2** shows the FTIR spectrum (Nicolet Thermo 6700c) comprising characteristic peaks at 3408, 3312, 3097, 2961, 2867, 2245, 1335, 1017, 760 and 617 cm⁻¹ wavenumbers. The Crystal modification 5 of (3R)-3-cyclopentyl-3-[4-(7H-pyrrolo[2,3-d]pyrimidin-4-yl)pyrazol-1-yl]propanenitrile hydrobromic acid salt is characterised by a Raman spectrum (Bruker RFS 100/S) comprising characteristic peaks at 3140, 3100, 3078, 2964, 2939, 2923, 2871, 2247, 1615 and 816 cm⁻¹ wavenumbers, shown in **Figure 3**.

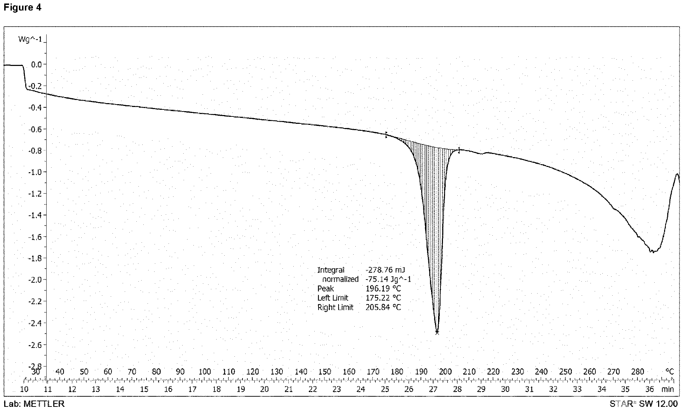

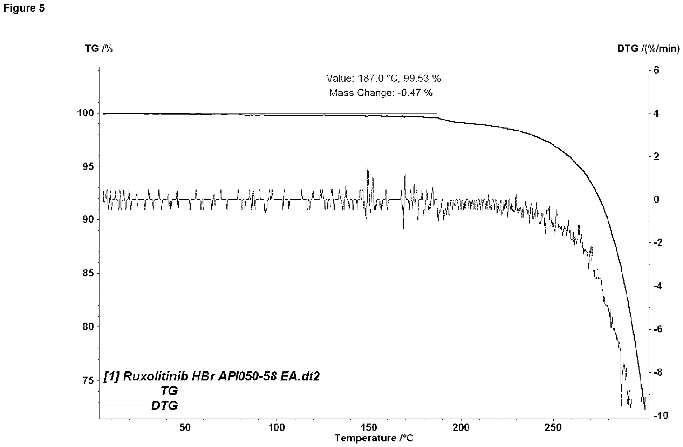

The Crystal modification 5 of (3R)-3-cyclopentyl-3-[4-(7H-pyrrolo[2,3-d]pyrimidin-4-yl)pyrazol-1-yl]propanenitrile hydrobromic acid salt can be further described by thermal analytical methods. **Figure 4** shows the DSC (Mettler-Toledo 822e DSC) and **Figure 5** shows the TGA (NETZSCH TG 209 thermogravimetric analyser) curves measured in the range of 25°C to 300°C and 25°C to 300°C, respectively. The Crystal modification 5 of (3R)-3-cyclopentyl-3-[4-(7H-pyrrolo[2,3-d]pyrimidin-4-yl)pyrazol-1-yl]propanenitrile hydrobromic acid salt shows a 0.47% weight loss in the range of 25°C to 190°C. The DSC measurement gives a melting process with Tₒₙₛₑₜ=175.2°C and Tpₑₐₖ=196.2°C.

The Crystal modification 5 of ruxolitinib hydrobromic acid salt can be prepared by a process comprising the steps of:
a) dissolution of ruxolitinib free base in a suitable organic solvent;
b) drop-wise addition of the hydrobromic acid 48% aqueous solution;
c) stirring the solution of the step b) at 50°C for additional 1 hour;
d) cooling the solution of the step c) to room temperature;
e) keeping the solution of the step d) for 16 hours at room temperature while precipitation occurred;
f) isolating the ruxolitinib hydrobromic acid salt in Crystal modification 5;
g) optionally, drying of the product of step f) at laboratory condition until the constant weight of the product is reached.

The suitable organic solvent is preferably a polar aprotic solvent, more preferably the polar aprotic solvent is selected from the group consisting of: acetone, acetonitrile, ethyl acetate, butyl acetate, tetrahydrofuran or a mixture thereof, even more preferably the suitable organic solvent is acetonitrile, even more preferably the suitable organic solvent is ethyl acetate at the temperature of 50°C.

Another process for the preparation of the Crystal modification 5 of ruxolitinib hydrobromic acid salt comprises the steps of:
a) suspending ruxolitinib hydrobromic acid salt in ethyl acetate at room temperature;
b) stirring the suspension of step a) at room temperature for 6 weeks;
c) isolating the Crystal modification 5 of ruxolitinib hydrobromic acid salt;
d) optionally, drying of the product of step c) at laboratory condition until the constant weight of the product is reached.

### Analysis - XRPD (X-Ray Powder Diffractometry)

Diffractograms were obtained with laboratory X'PERT PRO MPD PANalytical diffractometer, used radiation CuKα (λ = 1.542 Å).

Generator settings:
- excitation voltage 45 kV
- anodic current 40 mA.

Scan description:
- scan type - gonio
- measurement range 2 - 40° 2θ
- step size 0.01° 2θ
- step time: 0.5 s.

Samples were measured as received on Si plate (zero background holder).

Incident beam optics: programmable divergence slits (irradiated length 10 mm). 10 mm mask. 1/4° anti-scatter fixed slit, 0.02 rad Soller slits.

Diffracted beam optics: X'Celerator detector, scanning mode, active length 2.122°. 0.02 rad Soller slits, anti-scatter slit 5.0 mm. Ni filter.

### Analysis - FTIR (Fourier-Transformed Infra-Red) spectroscopy

FTIR spectra were recorded by Nicolet Thermo 6700 spectrometer.

### General settings:

Number of sample scans: 45
Number of background scans: 45
Resolution: 4.000
Sample gain: 4.0
Optical velocity: 0.6329
Aperture: 100.00

### Analysis - Raman spectroscopy

FTIR spectra were recorded by FT-Raman Bruker RFS 100/S Spectrometer

### General settings:

Excitation source: Nd-YAG laser (1064 nm)
Applied spectral domain: 4000-200 cm⁻¹
Applied laser power: 250 mW
Detector: liquid nitrogen cooled Ge-diode detector (D418-T)
Resolution: 4 cm⁻¹
Number of accumulations: 128
Scattering geometry: 180° (back scattering)
Aperture: 3.5 mm

### Analysis - DSC (Differential Scanning Calorimetry)

DSC measurements were performed using a Mettler-Toledo 822e DSC.

Samples were placed into standard aluminum pans (40 µL) sealed with a pierced lid. The sample cell was heated under a nitrogen purge at a rate of 10°C/min from 25°C up to a final temperature of 300°C with 50 mL/min nitrogen purge. The temperatures specified in relation to DSC analyses are the temperatures of the peak maxima (Tₚₑₐₖ) and onset temperature (Tₒₙₛₑₜ) of peaks for the crystalline form. The enthalpy is given in J/g.

The weight sample was about 2.5-3 mg.

### Analysis - TGA (ThermoGravimetric Analysis)

TGA analyses were performed using a NETZSCH TG 209 thermogravimetric analyser (NETZSCH-Gerätebau GmbH, Germany).

Each sample was placed in an aluminum sample pan and inserted into the TG furnace. The furnace was heated under nitrogen purge at a rate of 10°C/min from 25°C up to a final temperature of 300°C.

The weight sample was about 5-15 mg.

### Examples

The following examples are intended to further illustrate the present invention without limiting its scope.

### Example 1

### Preparation of Crystal modification 5 of (3R)-3-cyclopentyl-3-[4-(7H-pyrrolo[2,3-d]pyrimidin-4-yl)pyrazol-1-yl]propanenitrile hydrobromic acid salt

1 g (3.264 mmol) of ruxolitinib free base was dissolved in 2.5 mL of acetonitrile at 50°C applying a continuous stirring.

0.41 mL of hydrobromic acid (3.590 mmol; 48% aqueous solution) was drop-wise added to the acetonitrile solution of ruxolitinib at 50°C, while continuously stirred.

The suspension was further stirred at 50°C for additional 1 hour and then cooled back to room temperature. The solution was left to stir at that temperature overnight while precipitation occurred.

The solid precipitated is collected by filtration and dried at laboratory condition.
Product: 0.58 g (1.500 mmol) off-white crystalline solid
Yield: 46%
HPLC: 97.7%

XRPD pattern was measured (**Figure 1**) and showed that the compound is in a crystalline state that was designated as Crystal modification 5 of ruxolitinib hydrobromic acid salt.

### Example 2

### Preparation of Crystal modification 5 of (3R)-3-cyclopentyl-3-[4-(7H-pyrrolo[2,3-d]pyrimidin-4-yl)pyrazol-1-yl]propanenitrile hydrobromic acid salt

400 mg (1.033 mmol) of ruxolitinib hydrobromic acid salt was suspended in 0.8 mL of ethyl acetate at room temperature.

The suspension was kept in closed vessel at room temperature applying a continuous stirring for 6 weeks.

The solid obtained was collected by filtration and dried by vacuum suction at laboratory condition.

XRPD pattern was measured and showed that the compound is in a crystalline state that was designated as Crystal modification 5 of ruxolitinib hydrobromic acid salt.

## Claims

1. A crystal modification of a crystalline salt comprising (3R)-3-cyclopentyl-3-[4-(7H-pyrolo[2,3-d]pyrimidin-4-yl)pyrazol- 1--yl]propanenitrile of Formula I **characterized in that** the acid component HX is hydrobromic acid in a crystalline form having an X-ray powder diffraction pattern with characteristic peaks at about 4.3; 14.8; 18.9; 20.3; 22.5 and 25.6 ± 0.2° 2-theta.

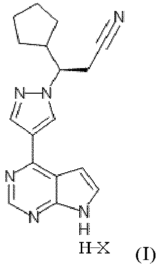

2. A process for the preparation of the Crystal modification of claim 1, comprising the steps of:
a) suspending ruxolitinib hydrobromic acid salt in ethyl acetate at room temperature;
b) stirring the suspension of step a) at room temperature for 6 weeks;
c) isolating the Crystal modification of ruxolitinib hydrobromic acid salt; and
d) optionally drying of the product of step c) at laboratory condition until the constant weight of the product is reached.

3. A pharmaceutical composition comprising a salt of (3R)-3-cyclopentyl-3-[4-(7H-pyrrolo[2,3-d]pyrimidin-4-yl)pyrazol-1-yl]propanenitrile of Formula I and acid component HX, **characterized in that** the acid component is hydrobromic acid in a crystalline form having an X-ray powder diffraction pattern with characteristic peaks at about 4.3; 14.8; 18.9; 20.3; 22.5 and 25.6 ± 0.2° 2-theta.

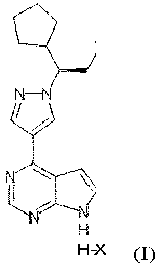

4. The pharmaceutical composition as claimed in claim 3, further comprising one or more pharmaceutically acceptable carriers or excipients.

5. The pharmaceutical composition as claimed in claims 3 to 4, which is in a pharmaceutical form suitable for oral administration.

6. The pharmaceutical composition of claims 3 to 5, wherein the pharmaceutical composition is in a form of a tablet.

## Patentansprüche

1. Kristallmodifikation eines kristallinen Salzes, das (3R)-3-Cyclopentyl-3-[4-(7H-pyrrolo[2,3-d]pyrimidin-4-yl)pyrazol-1-yl]propannitril von Formel I umfasst, **dadurch gekennzeichnet, dass** die Säurekomponente HX Bromwasserstoffsäure in kristalliner Form mit einem Röntgenpulverbeugungsmuster mit charakteristischen Peaks bei etwa 4,3; 14,8; 18,9; 20,3; 22,5 und 25,6 ± 0,2° 2-Theta ist.

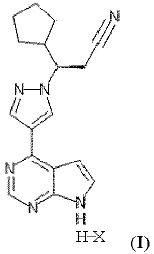

2. Verfahren zur Herstellung der Kristallmodifikation nach Anspruch 1, das die folgenden Schritte beinhaltet:
a) Suspendieren des Ruxolitinib-Bromwasserstoffsäuresalzes in Ethylacetat bei Raumtemperatur;
b) Rühren der Suspension aus Schritt a) bei Raumtemperatur für 6 Wochen;
c) Isolieren der Kristallmodifikation des Ruxolitinib-Bromwasserstoffsäuresalzes; und
d) optional Trocknen des Produkts aus Schritt c) unter Laborbedingungen, bis das konstante Gewicht des Produkts erreicht ist.

3. Pharmazeutische Zusammensetzung, die ein Salz von (3R)-3-Cyclopentyl-3-[4-(7H-pyrrolo[2,3-d]pyrimidin-4-yl)pyrazol-1-yl]propannitril von Formel I und die Säurekomponente HX umfasst, **dadurch gekennzeichnet, dass** die Säurekomponente Bromwasserstoffsäure in kristalliner Form mit einem Röntgenpulverbeugungsmuster mit charakteristischen Peaks bei etwa 4,3; 14,8; 18,9; 20,3; 22,5 und 25,6 ± 0,2° 2-Theta ist.

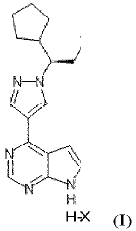

4. Pharmazeutische Zusammensetzung nach Anspruch 3, die ferner einen oder mehrere pharmazeutisch akzeptable Träger oder Hilfsstoffe umfasst.

5. Pharmazeutische Zusammensetzung nach den Ansprüchen 3 bis 4, die in zur oralen Verabreichung geeigneter pharmazeutischer Form vorliegt.

6. Pharmazeutische Zusammensetzung nach den Ansprüchen 3 bis 5, wobei die pharmazeutische Zusammensetzung in Form einer Tablette vorliegt.

## Revendications

1. Modification de forme cristalline d'un sel cristallin comprenant (3R)-3-cyclopentyl-3-[4-(7H-pyrolo[2,3-d]pyrimidin-4-yl)pyrazol-1-yl]propanenitrile de Formula I **caractérisée en ce que** le constituant acide HX est l'acide bromhydrique sous forme cristalline dont le diffractogramme des rayons X sur poudre présente des pics caractéristiques à environ 4,3 ; 14,8 ; 18,9 ; 20,3 ; 22,5 et 25, 6 ± 0.2° 2-thêta.

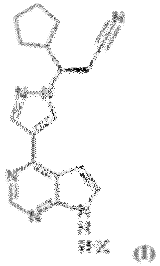

2. Processus de la modification de forme cristalline selon la revendication 1, comprenant les étapes suivantes :
a) la mise en suspension d'un sel d'acide bromhydrique de ruxolitinib dans de l'acétate d'éthyle à température ambiante ;
b) l'agitation de la suspension de l'étape a) à température ambiante pendant 6 semaines ;
c) l'isolation de la modification cristalline du sel d'acide bromhydrique de ruxolitinib ; et
d) le séchage facultatif du produit de l'étape c) en conditions de laboratoire jusqu'à obtention du poids constant du produit.

3. Composition pharmaceutique comprenant un sel de (3R)-3-cyclopentyl-3-[4-(7H-pyrolo[2,3-d]pyrimidin-4-yl)pyrazol-1-yl]propanenitrile de Formule I et un constituant acide HX, **caractérisée en ce que** le constituant acide et de l'acide bromhydrique de forme cristalline dont le diffractogramme des rayons X sur poudre présente des pics caractéristiques à environ 4,3 ; 14,8 ; 18,9 ; 20,3 ; 22,5 et 25, 6 ± 0.2° 2-thêta.

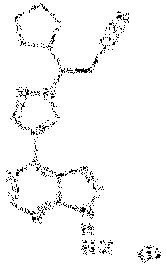

4. Composition pharmaceutique selon la revendication 3, comprenant en outre un ou plusieurs supports ou excipients pharmaceutiquement acceptables.

5. Composition pharmaceutique selon les revendications 3 à 4, laquelle se présente sous une forme pharmaceutique adaptée pour une administration orale.

6. Composition pharmaceutique selon les revendications 3 à 5, la composition pharmaceutique se présentant sous forme de cachet.
